Europäisches Patentamt

**European Patent Office**

**Office européen des brevets**

(11) Numéro de publication: **0 309 633 B1**

# (12) FASCICULE DE BREVET EUROPEEN

(45) Date de publication de fascicule du brevet: **01.04.92** (51) Int. Cl.⁵: **A61M 16/00**

(21) Numéro de dépôt: **87420261.7**

(22) Date de dépôt: **30.09.87**

(54) **Système d'alarme à commande pneumatique pour respirateur pneumatique.**

(43) Date de publication de la demande:
**05.04.89 Bulletin 89/14**

(45) Mention de la délivrance du brevet:
**01.04.92 Bulletin 92/14**

(84) Etats contractants désignés:
**AT BE CH DE ES GB GR IT LI LU NL SE**

(56) Documents cités:
**FR-A- 2 203 481**
**GB-A- 817 289**
**GB-A- 1 073 704**
**US-A- 4 155 357**

(73) Titulaire: **DRAGER**
**60 Rue de la Glacière**
**F-75013 Paris(FR)**

(72) Inventeur: **Le Bec, Yves**
**Ecole Jean Perrin Avenue des Tuileries**
**F-91350 Grigny(FR)**

(74) Mandataire: **Maureau, Philippe et al**
**Cabinet Germain & Maureau Le Britannia -**
**Tour C 20, bld Eugène Déruelle**
**F-69003 Lyon(FR)**

# Description

Il est habituel d'associer à un respirateur pneumatique un système d'alarme sonore et/ou visuelle qui intervient lorsque le respirateur ne remplit plus sa fonction. Il en est ainsi notamment :

- en cas de débranchement du patient,
- en cas de chute de pression de la source de gaz respiratoire,
- en cas de panne du respirateur,
- et en cas d'alimentation en un mélange gazeux respiratoire dont le taux d'oxygène est inférieur à la limite admissible.

Les systèmes d'alarme peuvent être divisés en deux types : ceux électriques ou électroniques, et ceux pneumatiques.

Les systèmes d'alarme électriques ou électroniques ne présentent pas d'inconvénients majeurs lorsqu'il s'agit de respirateurs associés à des installations fixes, ce qui est le cas dans les blocs opératoires. Par contre, pour être utilisés avec des installations mobiles, par exemple ambulances, ces systèmes nécessitent une énergie électrique fournie par des piles ou des accumulateurs. Or, il s'avère que les piles ou les accumulateurs ne sont pas toujours en état de fonctionnement ; et de plus, de telles sources d'énergie électrique ne sont pas utilisables dans certaines atmosphères, par exemple en atmosphère déflagrante.

Quant aux systèmes d'alarme à commande pneumatique, ils sont généralement complexes, et en tout cas, ils ne sont pas utilisables sur des respirateurs transportables car leur consommation en gaz est trop élevée.

Les brevets français N° 2 203 481 et britannique N° 817 289 décrivent des systèmes d'alarme pneumatique, très différents de celui faisant l'objet de ladite revendication 1 puisqu'ils comprennent deux circuits de gaz dont un est principal et l'autre secondaire et que le fonctionnement de l'alarme nécessite l'emploi de deux gaz, le gaz du circuit secondaire (protoxyde d'azote) provoquant le déclenchement de l'alarme en fonction de la pression régnant dans le circuit.

Ces dispositifs présentent l'inconvénient qu'en cas de défaillance dans le circuit secondaire, l'alarme n'est pas déclenchée même si une défaillance survient dans le circuit principal, contenant l'oxygène.

L'invention vise à remédier à ces inconvénients, en proposant un système d'alarme à commande entièrement pneumatique qui, associé à un respirateur pneumatique, est utilisable sur toute installation fixe ou mobile, présente un caractère de grande simplicité et ne nécessite qu'une consommation très réduite de gaz.

Conformément à l'invention, sur le circuit reliant le respirateur au patient, est branchée une dérivation comprenant une vanne du type à ouverture brusque qui est maintenue en position d'ouverture par un ressort et qui, dans sa position d'ouverture, alimente en gaz un système d'alarme sonore fonctionnant pneumatiquement, cette vanne étant soumise à un signal de pilotage pour commander, en fonction de la pression régnant dans le circuit reliant le respirateur pneumatique au patient, soite a fermeture de cette vanne et l'arrêt de l'alarme sonore, soit au contraire l'ouverture de cette vanne et le fonctionnement de l'alarme.

Suivant une forme particulière d'exécution, le système d'alarme sonore consiste en un réservoir qui est branché sur l'orifice de sortie de la vanne et est divisé par une membrane en deux chambres reliées à la source de gaz provenant de la vanne, l'une par une canalisation contenant une résistance fixe, et l'autre par une canalisation contenant une résistance variable, cette dernière chambre comportant un orifice de mise à l'air libre. C'est la vibration de cette membrane qui constitue le signal sonore ; son intensité est réglable en agissant sur la résistance variable prévue sur la canalisation alimentant la chambre communiquant avec l'air libre.

Pour permettre le fonctionnement de ce système d'alarme à commande pneumatique dans tous les cas, et plus particulièrement non exclusivement lorsqu'il y a débranchement du patient, il est prévu, selon l'invention, un relais amplificateur branché sur le circuit reliant le respirateur au patient, et alimenté par une source de gaz sous pression, et ce relais est relié, avec interposition d'un clapet anti-retour, à un réservoir qui est raccordé à la vanne commandant l'alarme sonore et dont le gaz sous pression constitue le moyen de commande de fermeture de cette vanne qui comporte deux autres orifices pour son raccordement respectivement à une source de gaz sous pression et au signal sonore.

En outre, et c'est là une autre caractéristique de l'invention, pour permettre à ce système d'alarme à commande pneumatique d'être efficace dans toutes les conditions d'utilisation, il est complété par un signal visuel consistant en un voyant pneumatique avec fonction logique "inhibition" qui est raccordé d'une part, au réservoir à gaz commandant la fermeture de la vanne contrôlant le fonctionnement de l'alarme sonore, et d'autre part au circuit reliant le patient au respirateur.

L'invention sera bien comprise d'ailleurs et ses avantages, ainsi que d'autres caractéristiques ressortiront de la description qui suit, en référence au dessin schématique annexé représentant, à titre d'exemples non limitatifs, quelques formes d'exécution de ce système d'alarme à commande pneumatique pour respirateur :

Figure 1 est un schéma de montage du système le plus simple représenté en position normale d'alarme sonore ;

Figure 2 montre le même système en position d'utilisation, mais hors fonctionnement de l'alarme ;

Figures 3 et 4 sont des vues identiques aux figures 1 et 2 dans le cas d'un système dans lequel la vanne est commandée par un relais amplificateur ;

Figures 5 et 6 sont des vues identiques aux figures 3 et 4 dans le cas d'un système dans lequel une alarme visuelle est associée à l'alarme sonore ;

Figure 7 illustre un système d'alarme identique à celui de figures 5 et 6, mais dans lequel un moyen de commande manuelle est prévu pour mettre volontairement hors service le signal sonore.

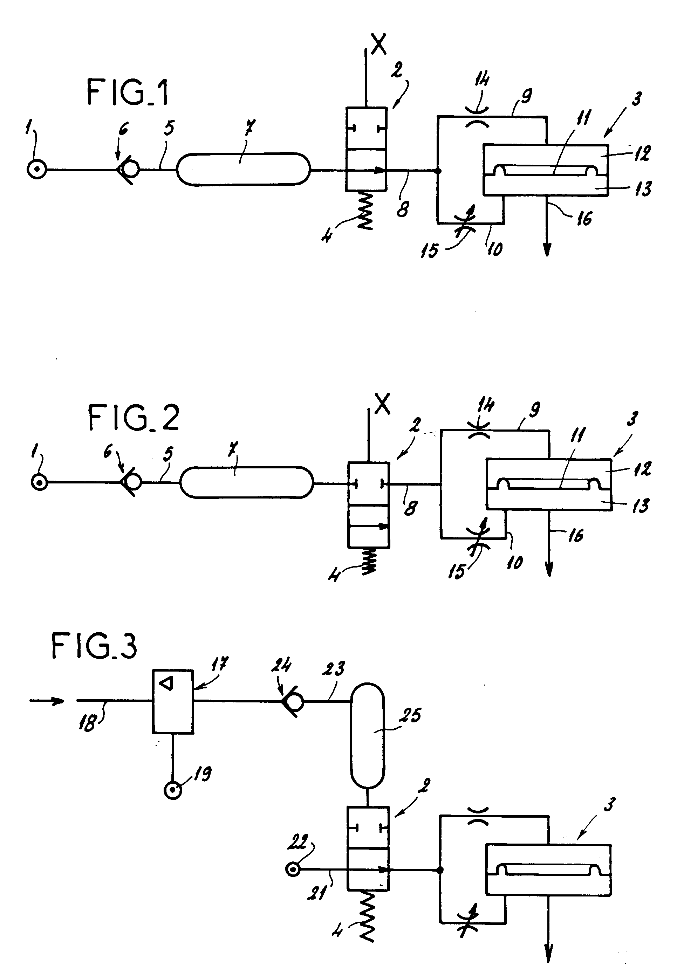

En référence aux figures 1 et 2, 1 désigne la source d'alimentation en gaz sous pression ; il peut s'agir d'une bouteille portative ou du réseau d'une installation fixe. 2 désigne une vanne dont la position normale est celle d'ouverture ; ce type de vanne est généralement désigné par le sigle "NO". 3 désigne de façon générale une alarme sonore.

Conformément à l'invention, la vanne 2 soumise à l'action d'un ressort 4 tendant à la maintenir en position d'ouverture est asservie à un signal de pilotage dont le fonctionnement est contrôlé par exemple par la pression régnant dans le circuit reliant le patient au respirateur ou par la pression du gaz respiratoire dans le mélangeur de sécurité habituellement utilisé. Cette vanne 2 est reliée à la source 1 d'alimentation en gaz par une canalisation 5 comportant d'amont en aval un clapet anti-retour 6 et un petit réservoir 7. La vanne 2 est reliée non seulement à ce réservoir 7, mais aussi à l'alarme sonore 3 par une canalisation 8 présentant deux dérivations respectivement 9 et 10 aboutissant dans le corps de l'alarme qui est divisé en deux chambres 12 et 13 par une membrane 11. Dans la chambre 12 débouche la canalisation 9 qui contient une résistance fixe 14. Dans la chambre 13 débouche la canalisation 10 qui contient une résistance variable 15 ; et cette chambre 13 comporte un tube de mise à l'air libre 16.

Lorsque le système est en position de repos, la vanne 2 occupe la position d'ouverture représentée à la figure 1. Dans cette position, l'alarme 3 est alimentée au travers de la vanne 2 par le réservoir 7 relié à la source 1 d'alimentation en gaz. Le gaz pénétrant dans le corps de l'alarme par les canalisations 9 et 10 entraîne une vibration de la membrane 11 ; et le niveau sonore de cette vibration est réglable par action manuelle sur la résistance 15.

Lorsque le respirateur est en position normale de fonctionnement, le signal de pilotage X qui en résulte provoque la fermeture de la vanne 2 en l'amenant dans la position représentée à la figure 2. L'alarme 3 n'étant alors plus reliée au réservoir 7 se trouve mise hors circuit. Ce réservoir demeure toutefois sous pression, de telle sorte que, si le signal de pilotage commandant la vanne 2 cesse, le ressort 4 ramène instantanément et brusquement la vanne en position d'ouverture, ce qui entraîne à nouveau, grâce au gaz contenu dans le réservoir 7, le fonctionnement de l'alarme sonore 3.

Il est à noter que le réservoir 7 peut être alimenté ou non par la même source de gaz que le respirateur branché sur le patient. Si l'alimentation est la même, le système peut être entièrement autonome.

Il est également à noter que la durée de fonctionnement du signal sonore 3 est de l'ordre de 7 à 10 secondes en cas de coupure complète de l'alimentation du réservoir 7 en gaz, mais que si la chute de pression est progressive, la durée de fonctionnement du signal est beaucoup plus longue.

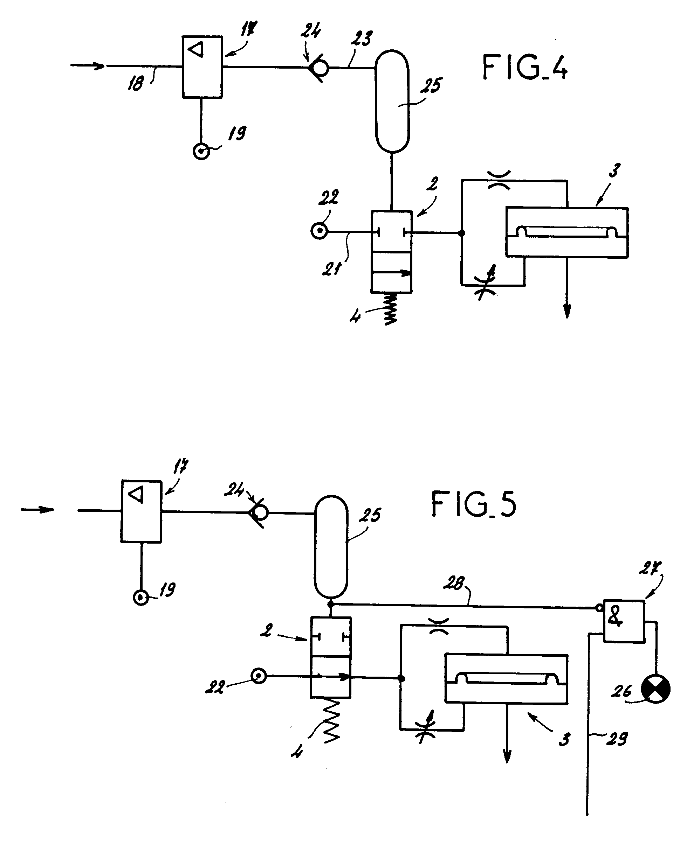

Les figures 3 et 4 représentent un système du même genre, mais dans lequel la vanne 2 (vanne NO) est commandée par un relais amplificateur 17 piloté à très faible pression.

Le signal de pilotage provient du branchement du relais 17 par une canalisation 18 sur le circuit reliant le patient au respirateur ; et le relais amplificateur 17 est relié par ailleurs directement à la source 19 d'alimentation en gaz, la pression d'alimentation étant généralement comprise entre 1 et 10 bars.

La vanne 2 maintenue normalement en position d'ouverture par son ressort de rappel 4 est reliée, d'une part, directement par une canalisation 21 à une source d'alimentation en gaz 22 qui peut être identique ou différente de la source 19. La vanne 2 est d'autre part reliée, comme dans le cas précédent, à l'alarme sonore 3.

Le signal de pilotage à très faible pression étant produit par la pression régnant dans le circuit qui relie le patient au respirateur, chaque insufflation entraîne une augmentation de la pression et commande ainsi le relais amplificateur 17 qui délivre un signal amplifié dont la pression est par exemple de 1,5 bar. Ce signal provoque la fermeture de la vanne 2 et supprime ainsi le fonctionnement du signal sonore d'alarme, car le relais amplificateur 17 est relié à la vanne 2 par une canalisation 23 comportant un clapet anti-retour 24, et par un réservoir 25 branché sur la vanne 2.

Pendant la phase expiratoire, le relais amplificateur 17 est relâché, et le réservoir 25 se purge très lentement par un débit inverse du clapet anti-retour 24 qui oppose en effet une très grande résistance.

A chaque insufflation, le système est réactivé ; et il faut plus d'une dizaine de secondes sans réactivation pour que la vanne 2 s'ouvre brusquement sous l'effet de son ressort de rappel 4 en entraînant le fonctionnement de l'alarme sonore. Il en est ainsi notamment si le circuit reliant le patient au respirateur n'enregistre plus de montée en pression ; c'est le cas notamment s'il y a débranchement du patient.

Ce système possède, entre autres avantages :
- celui de permettre un test automatique de l'alarme lors de la mise en marche de l'installation, car l'alarme fonctionne jusqu'à ce que la première inspiration vienne l'arrêter,
- et celui de déclenchement de l'alarme non seulement en cas de débranchement du malade, mais aussi en cas de chute de pression dans l'installation d'alimentation en gaz, notamment par exemple s'il s'agit d'une bouteille.

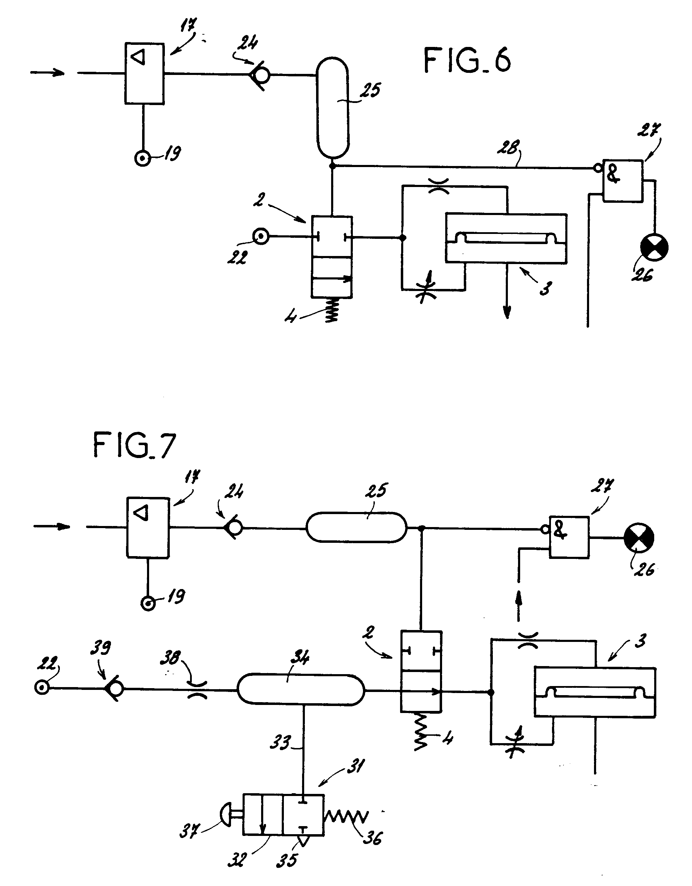

Le système représenté aux figures 5 et 6 est identique à celui de figures 3 et 4, mais est perfectionné en ce sens qu'à l'alarme sonore 3 est associée une alarme visuelle. Cette alarme est constituée par un voyant pneumatique 26 avec fonction logique "inhibition" 27, branché d'une part par une canalisation 28 sur ou à la sortie du réservoir 25, et branché d'autre part par une canalisation 29 sur la commande d'inspiration.

Lorsque la pression dans le réservoir 25 est suffisamment élevée, le voyant 26 est inhibé. Par contre, lorsque la pression chute, l'inhibition disparaît, et le voyant 26 est alors actionné au même rythme que le respirateur. Il en est ainsi par exemple en cas de débranchement du patient.

Ce système perfectionné présente, entre autres, les avantages ci-après :
- l'alarme visuelle 26 remplit son rôle dans toutes les circonstances, même dans un environnement bruyant, par exemple à l'intérieur d'un hélicoptère, alors que l'alarme sonore est inaudible,
- le fonctionnement du voyant 26 ne nécessite aucune consommation de gaz,
- et, comme dans le cas précédent (figures 3 et 4), il y a test automatique du système d'alarme pendant la première inspiration lors de la mise en marche de l'installation.

Le système représenté à la figure 7 ne diffère de celui illustré par les figures 5 et 6 que par l'adjonction d'un organe supplémentaire. Il s'agit d'un bouton de commande 31 qui permet à volonté de mettre hors service l'alarme sonore 3. Cet organe 31 est constitué par une vanne 32 qui est raccordée par une canalisation 33 à un réservoir 34 et qui comporte un orifice de purge 35. Cette vanne 32 est soumise à l'action d'un ressort 36 tendant à la maintenir en position de fermeture ; et elle comporte un bouton-poussoir 37 permettant l'ouverture manuelle.

Le réservoir 34 est interposé entre la vanne 2 (vanne NO) et la source d'alimentation en gaz 22 ; et en amont du réservoir 34 sont prévus une résistance 38 et un clapet anti-retour 39.

En position normale, la vanne 32 est fermée ; l'alarme sonore 3 et le voyant 26 fonctionnent donc comme dans le cas précédent (figures 5 et 6), l'alimentation de l'alarme sonore 3 étant faite au travers de la vanne 2 par le réservoir 34. L'alarme sonore 3 et le voyant 26 fonctionnent ainsi normalement en cas de débranchement du patient ou en cas de chute de pression ; mais une action sur le bouton-poussoir 37 permet de purger immédiatement le réservoir 34. L'alarme sonore 3 cesse donc de fonctionner ; mais l'alarme visuelle 26 continue à fonctionner. Il suffit de relâcher le bouton-poussoir 37 pour que le réservoir 34 se remplisse à nouveau à travers la résistance 38 qui est plus faible que celle de l'alarme sonore 3 et pour qu'ainsi au bout de quelques instants, l'alarme sonore 3 fonctionne à nouveau.

Comme il va de soi, l'invention ne se limite pas aux seules formes d'exécution de ce système d'alarme à commande pneumatique qui ont été ci-dessus indiquées à titre d'exemples ; elle en embrasse, au contraire, toutes les variantes de réalisation et d'application.

**Revendications**

1. Système d'alarme à commande pneumatique pour respirateur pneumatique, caractérisé en ce que, sur le circuit (1, 22) reliant le respirateur au patient, est branchée une dérivation comprenant une vanne (2) du type à ouverture brusque qui est maintenue en position d'ouverture par un ressort (4) et qui, dans sa position d'ouverture, alimente en gaz d'un système d'alarme sonore (3) fonctionnant pneumatiquement, cette vanne (2) étant soumise a un signal de pilotage (X) pour commander, en fonction de la pression régnant dans le circuit reliant le respirateur pneumatique au patient, soit la fermeture de cette vanne (2) et l'arrêt de l'alarme sonore (3), soit au contraire l'ouverture de cette vanne (2) et le fonctionnement de l'alarme (3), un réservoir (7) de gaz rempli à travers un clapet anti-retour (6), qui relie la vanne (2) à la source d'alimentation (1) en gaz, étant, en outre, prévu pour provoquer le fonctionnement de l'alarme sonore (3) lors de l'ouverture de la vanne (2).

2. Système d'alarme à commande pneumatique selon la revendication 1, caractérisé en ce que son système d'alarme sonore est constitué par un réservoir qui est branché sur l'orifice de sortie de la vanne (2) et est divisé par une membrane (11) en deux chambres (12,13) reliées à la source de gaz provenant de la vanne (2), l'une (12) par une canalisation (9) contenant une résistance fixe (14), et l'autre (13) par une canalisation (10) contenant une résistance variable (15), cette dernière chambre (13) comportant un orifice de mise à l'air libre (16).

3. Système d'alarme à commande pneumatique selon les revendications 1 et 2, caractérisé en ce qu'un relais amplificateur (17) est branché sur le circuit reliant le respirateur au patient, est alimenté par une source de gaz sous pression, et est relié avec interposition d'un clapet anti-retour (24), à un réservoir (25) qui est raccordé à la vanne (2) commandant l'alarme sonore (3) et dont le gaz sous pression constitue le moyen de commande de fermeture de cette vanne qui comporte deux autres orifices pour son raccordement respectivement à une source (22) de gaz sous pression et au signal sonore (3).

4. Système d'alarme à commande pneumatique selon l'une quelconque des revendications 1 à 3, caractérisé en ce qu'il est complété par un signal visuel consistant en un voyant pneumatique (26) avec fonction logique "inhibition" (27) qui est raccordé d'une part, au réservoir à gaz (25) commandant la fermeture de la vanne (2) contrôlant le fonctionnement de l'alarme sonore (3), et d'autre part, au circuit reliant le patient au respirateur.

5. Système d'alarme à commande pneumatique selon l'une quelconque des revendications 1 à 4, caractérisé en ce que l'alarme sonore (3) est alimentée au travers de la vanne (2) par un réservoir (34) relié à la source de gaz sous pression par l'intermédiaire d'une résistance (38) et d'un clapet anti-retour (39), et ce réservoir (34) est relié à une deuxième vanne (31) qui débouche à l'air libre, est soumise à l'action d'un ressort (36) tendant à la maintenir en position de fermeture et porte un bouton-poussoir (37) permettant son ouverture pour la mise à l'air libre du réservoir (34).

**Claims**

1. A pneumatically controlled alarm system for a pneumatic respirator, characterised in that, on the circuit (1, 22) connecting the respirator to the patient, there is connected a branch comprising a valve (2) of the rapid opening type which is held in open position by a spring (4) and which, in its open position, supplies with gas a pneumatically operating audible alarm system (3), the valve (2) being subjected to a pilot signal (X) for controlling either the closure of the valve (2) and the stopping of the audible alarm (3), or conversely the opening of the valve (2) and operation of the alarm (3) as a function of the pressure prevailing in the circuit connecting the pneumatic respirator to the patient, a gas reservoir (7) filled through a non-return valve (6), which connects the valve (2) to the gas supply source (1), being, in addition, provided to cause the operation of the audible alarm (3) when the valve (2) opens.

2. A pneumatically controlled alarm system according to Claim 1, characterised in that its audible alarm system is constituted by a reservoir which is connected to the output orifice of the valve (2) and is divided by a membrane (11) into two chambers (12, 13) connected to the source of gas issuing from the valve (2), the one (12) by a passage (9) containing a fixed resistance (14), and the other (13) by a passage (10) containing a variable resistance (15), this latter chamber (13) having an orifice (16) connected to atmosphere.

3. A pneumatically controlled alarm system according to Claims 1 and 2, characterised in that an amplifier relay (17) is connected in the circuit connecting the respirator to the patient, is supplied by a source of gas under pressure, and is connected, with the interposition of a non-return valve (24), to a reservoir (25) which is connected to the valve (2) controlling the audible alarm (3) and of which the gas under pressure constitutes the means for controlling the closure of this valve which includes two other orifices for its connection respectively to a source (22) of gas under pressure and to the audible alarm (3).

4. A pneumatically controlled alarm system according to any one of Claims 1 to 3, characterised in that it is completed by a visual signal consisting of a pneumatic indicator (26) with an 'inhibition' logic function (27) which is connected, on the one hand, to the gas reservoir (25) controlling the closure of the valve (2) which controls the operation of the audible alarm (3) and, on the other hand, to the circuit connecting the patient to the respirator.

5. A pneumatically controlled alarm system according to any one of Claims 1 to 4, characterised in that the audible alarm (3) is supplied through the valve (2) by a reservoir (34) connected to the source of gas under pressure by means of a resistance (38) and a non-return valve (39), and this reservoir (34) is connected to a second valve (31) which opens into free atmosphere, is subjected to the action of a spring (36) tending to hold it in the closed position and carries a push-button (37) permitting its opening to connect the reservoir (34) to atmosphere.

## Patentansprüche

1. Alarmsystem mit pneumatischer Steuerung für ein pneumatisches Beatmungsgerät, **dadurch gekennzeichnet,** daß von dem Kreislauf (1, 22) der das Beatmungsgerät mit dem Patienten verbindet, eine Zweigleitung abgezweigt ist, die ein Schnellschaltventil (2) welches mit Hilfe einer Feder (4) in Durchlaßstellung gehalten wird und welches ein pneumatisch funktionierendes akustisches Alarmsystem (3) mit Gas versorgt, beinhaltet, wobei das Ventil (2) durch ein Steuersignal x beaufschlagt wird, um abhängig von dem Druck, der in dem Kreislauf zwischen Beatmungsgerät und Patient herrscht, sei es das Schließen des Ventils (2) und das Unterbrechen des akustischen Alarms (3), sei es im Gegenteil das Öffnen des Ventils (2) und das Ingangsetzen des Alarms (3) zu steuern, wobei ein durch ein Rückschlagventil (6) gefüllter Gasspeicher (7) vorgesehen ist, der das Ventil (2) mit der Versorgungsquelle (1) verbindet und der die Funktion des akustischen Alarms bei Öffnen des Ventils (2) sicherstellt.

2. Alarmsystem mit pneumatischer Steuerung nach Anspruch 1, dadurch gekennzeichnet, daß sein akustisches Alarmsystem einen Speicher beinhaltet, der mit der Ausgangsöffnung des Ventiles (2) verbunden ist und der durch eine Membrane (11) in zwei Kammern (12, 13) geteilt wird, die unter Zwischenschaltung des Ventiles (2) mit der Gasquelle verbunden sind, wobei sich in der Leitung zwischen Ventil (2) und Kammer (12) ein fester Widerstand befindet, wohingegen sich in der Zuleitung zur Kammer (13), die eine Öffnung zur Umgebungsluft hat, ein regelbarer Widerstand (15) befindet.

3. Alarmsystem mit pneumatischer Steuerung nach den Ansprüchen 1 und 2, dadurch gekennzeichnet, daß mit dem Kreislauf zwischen Patient und Beatmungsgerät ein Verstärkungsrelais (17) verbunden ist, das von einer Druckgasquelle versorgt wird und der unter Zwischenschaltung eines Rückschlagventils (24) an einen Speicher (25) angeschlossen ist, der wiederum mit dem Ventil (2), welches den akustischen Alarm steuert, verbunden ist, wobei folglich dessen Gas das Steuermittel zum Schließen des Ventils bildet, das zwei weitere Anschlußöffnungen für eine Druckgasquelle und für das akustische Alarmsignal besitzt.

4. Alarmsystem mit pneumatischer Steuerung nach einem der Ansprüche 1 bis 3, dadurch gekennzeichnet, daß sie durch ein optisches Signal vervollständigt wird, das durch eine pneumatische Anzeigeeinrichtung (26) mit logischer Inhibitionsfunktion, die auf der einen Seite mit dem Gasspeicher (25), der das Schließen des Ventiles (2) veranlaßt, welches die Funktion des akustischen Alarms (3) steuert, und auf der anderen Seite mit dem Kreislauf zwischen Beatmungsgerät und Patient verbunden ist.

5. Alarmsystem mit pneumatischer Steuerung nach einem der Ansprüche 1 bis 4, dadurch gekennzeichnet, daß der akustische Alarm (3) über das Ventil (2) von einem Speicher (34) versorgt wird, der unter Zwischenschaltung eines Widerstandes (38) und eines Rückschlagventils (39) mit einer Druckgasquelle verbunden ist, wobei der Speicher (34) mit einem zweiten Ventil (31) in Verbindung steht, welches eine Öffnung zur Umgebungsluft besitzt und mit Hilfe einer Feder (36) in Schließstellung gehalten wird und welches einen Druckknopf trägt, der es ermöglicht, das Ventil zu öffnen, um dem Speicher (34) mit der Umgebungsluft zu verbinden.

FIG.1

FIG.2

FIG.3

FIG_4

FIG_5

FIG.6

FIG.7